(19) Europäisches Patentamt
European Patent Office
Office européen des brevets

(11) **EP 3 711 803 A1**

(12) **DEMANDE DE BREVET EUROPEEN**

(43) Date de publication:
**23.09.2020 Bulletin 2020/39**

(51) Int Cl.:
**A61M 16/00** *(2006.01)* **A61M 16/10** *(2006.01)*
**A61M 16/12** *(2006.01)* **A61M 16/20** *(2006.01)*
**B01D 53/00** *(2006.01)* **B01D 53/22** *(2006.01)*

(21) Numéro de dépôt: **20159190.6**

(22) Date de dépôt: **25.02.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **22.03.2019 FR 1902967**

(71) Demandeur: **L'AIR LIQUIDE, SOCIETE ANONYME POUR L'ETUDE ET L'EXPLOITATION DES PROCEDES GEORGES CLAUDE**
**75007 Paris (FR)**

(72) Inventeur: **BOULANGER, Thierry**
**Newark, DE Delaware 19702 (US)**

(74) Mandataire: **Pittis, Olivier**
**L'Air Liquide S.A.**
**75, quai d'Orsay**
**75321 Paris Cedex 07 (FR)**

Remarques:
Revendications modifiées conformément à la règle 137(2) CBE.

(54) **APPAREIL ET INSTALLATION DE FOURNITURE D'UN MÉLANGE GAZEUX À UN PATIENT**

(57) L'invention concerne un appareil (1) de fourniture d'un mélange gazeux à un patient comprenant un conduit d'entrée de gaz (30) comprenant un orifice d'entrée de gaz (30a) se divisant en un premier conduit de gaz (31) et en un deuxième conduit de gaz (32) ; au moins un module de perméation (33) agencé sur le deuxième conduit de gaz (32), ledit module de perméation (33) comprenant un port d'alimentation (33a) en communication fluidique avec le deuxième conduit de gaz (32), un port de rétentat (33b) et un port de perméat (33c) ; un troisième conduit de gaz (34) en communication fluidique avec le port de rétentat (33b) du module de perméation (33) ; un quatrième conduit de gaz (35) en communication fluidique avec le port de de perméat (33c) du module de perméation (33), et venant se raccorder fluidiquement audit premier conduit de gaz (31) ; et une source d'air (360) en communication fluidique avec le premier conduit de gaz (31) et le quatrième conduit de gaz (35). Installation (1, 2, 11) de réalisation et fourniture de mélange gazeux (1, 2, 11) comprenant un tel appareil (1) de fourniture de mélange gazeux, une source de gaz (2) contenant un prémélange gazeux à plusieurs constituants, de préférence un prémélange $N_2O/O_2$, et un circuit patient (11) avec une interface respiratoire (10).

[Fig. 1]

EP 3 711 803 A1

**Description**

La présente invention concerne un appareil et une installation de fourniture d'un mélange gazeux médical à un patient, notamment un mélange de gaz contenant du protoxyde d'azote et de l'oxygène, en particulier un mélange ternaire formé essentiellement de protoxyde d'azote, d'oxygène et d'azote.

Le protoxyde d'azote, $N_2O$, est un gaz thérapeutique qui possède des propriétés analgésiques (i.e. réduction de la douleur) et anxiolytiques (i.e. réduction du stress) avec un effet dans les minutes qui suivent l'administration et un retour aussi rapide à l'état initial, après interruption de l'administration. De plus, le $N_2O$ est sûr, a des effets secondaires limités et n'est pas métabolisé.

Il est généralement administré à une concentration élevée, par exemple environ 70 % équilibré avec 30% d'oxygène ($O_2$) (% molaires) au début d'une procédure d'anesthésie, avant l'introduction d'agents anesthésiques plus puissants. Dans ce cas, une source de $N_2O$ médical pur, provenant d'une bouteille ou d'une prise murale, est utilisée.

Plus communément, il est utilisé pour accompagner les accouchements dans les cliniques ou bien dans les cabinets dentaires pour faciliter les soins. Pour ces applications, qui nécessitent un effet analgésique moins puissant, une bouteille contenant un prémélange de qualité médicale composé de 50% de $N_2O$ et de 50% de $O_2$ (% molaires) est utilisée.

Or, l'administration de longue durée et répétée de ce type de prémélange peut poser problèmes.

En effet, la teneur élevée en oxygène de 50%mol. peut générer des cas répétés d'hyperoxie pouvant affecter négativement l'état du patient après traitement, par exemple si ledit patient souffre de comorbidité telle qu'une insuffisance cardiaque chronique ou une bronchopneumopathie chronique obstructive.

Idéalement, la concentration en oxygène doit alors être réglée au minimum pour éviter toute situation d'hypoxémie pouvant survenir pendant ou après la fin du traitement en raison des propriétés du $N_2O$, typiquement une concentration en oxygène généralement de l'ordre de 30%mol.

Or, réaliser un mélange gazeux contenant du $N_2O$ et une telle concentration d'oxygène n'est pas aussi simple qu'il n'y paraît.

Dès lors, le problème est de pouvoir réaliser un mélange ternaire contenant essentiellement $N_2O$, $O_2$ et $N_2$, en contrôlant la concentration en $O_2$, par exemple une teneur fixe de 30%mol., mais tout en offrant la possibilité de faire varier la concentration de $N_2O$, en limitant le nombre de sources de gaz sous pression utilisées, c'est-à-dire de bouteilles ou autres récipients de gaz, de préférence une seule bouteille ou analogue contenant un prémélange non hypoxique répondant aux exigences de déploiement à domicile, c'est-à-dire au domicile du patient.

Une solution de l'invention concerne alors un appareil de fourniture d'un mélange gazeux à un patient, aussi appelé générateur de mélange gazeux, comprenant :

- un conduit d'entrée de gaz comprenant un orifice d'entrée de gaz se divisant en un premier conduit de gaz et en un deuxième conduit de gaz,
- au moins un module de perméation agencé sur le deuxième conduit de gaz, ledit module de perméation comprenant un port d'alimentation en communication fluidique avec le deuxième conduit de gaz, un port de rétentat et un port de perméat,
- un troisième conduit de gaz en communication fluidique avec le port de rétentat du module de perméation,
- un quatrième conduit de gaz en communication fluidique avec le port de de perméat du module de perméation, et venant se raccorder fluidiquement audit premier conduit de gaz et
- une source d'air en communication fluidique avec le premier conduit de gaz et le quatrième conduit de gaz.

Dans le cadre de la présente invention :

- les pourcentages (%) sont des pourcentages molaires (%mol.),
- l'air est considéré comme formé d'un mélange d'oxygène (env. 20 à 21%) et d'azote (env. 78%), i.e. un mélange $O_2/N_2$. Les autres constituants éventuellement présents (Ar, CO2...) sont considérés négligeables (<2% env.) et traités comme étant des impuretés inévitables.

Selon le mode de réalisation considéré, l'appareil de fourniture de mélange gazeux selon l'invention peut comprendre l'une ou plusieurs des caractéristiques suivantes :

- le module de perméation comprend une pluralité de fibres creuses.
- les fibres creuses comprennent chacune un paroi périphérique formant membrane de perméation et un passage interne de gaz ou lumen.
- le module de perméation comprend plusieurs centaines de fibres creuses agencées en parallèles, de préférence au moins un millier de fibres creuses.

- le module de perméation comprend un port d'alimentation en gaz en communication fluidique avec le deuxième conduit et par lequel arrive le mélange gazeux d'alimentation provenant du deuxième conduit.
- le module de perméation comprend en outre un port de rétentat en communication fluidique avec le troisième conduit de gaz et d'où une partie du gaz d'alimentation ressort du module de perméation en étant retenu, c'est-à-dire d'où ressort le (ou les) composé qui ne permée pas au travers des membranes des fibres creuses.
- un port de perméat en communication fluidique avec le quatrième conduit de gaz et d'où une partie du gaz ayant perméé ressort du module de perméation, c'est-à-dire d'où ressort le (ou les) composé qui permée au travers des membranes des fibres creuses.
- le port d'alimentation est en communication fluidique avec une première chambre du module de perméation, aussi appelée chambre amont ou chambre d'alimentation.
- le module de perméation comprend par ailleurs une deuxième chambre, aussi appelée chambre aval ou chambre de rétentat, comprenant le port de rétentat.
- le module de perméation comprend par ailleurs une troisième chambre, aussi appelée chambre intermédiaire ou chambre de perméat, comprenant le port de perméat.
- les fibres creuses sont agencées principalement dans la troisième chambre.
- le premier conduit de gaz comprend une première électrovanne proportionnelle et un premier capteur de débit.
- la première électrovanne proportionnelle et le premier capteur de débit définissent ensemble un contrôleur de débit massique.
- la première électrovanne proportionnelle et le premier capteur de débit sont alimentés électriquement et contrôlés par une carte de commande électronique et pilotés un microcontrôleur de la carte de commande électronique.
- le deuxième conduit de gaz comprend une deuxième électrovanne proportionnelle.
- le troisième conduit de gaz comprend un capteur de pression et une troisième électrovanne proportionnelle, c'est-à-dire que le capteur de pression a sa prise de pression reliée fluidiquement au troisième conduit de gaz.
- le quatrième conduit de gaz comprend un deuxième capteur de débit et un premier capteur d'oxygène.
- le premier capteur de débit et/ou le deuxième capteur de débit sont des capteurs de débit massique.
- la source d'air comprend une pompe à air reliée fluidiquement à l'atmosphère.
- il comprend en outre une unité de commande à microcontrôleur.
- l'unité de commande à microcontrôleur comprend la carte électronique, de préférence le microcontrôleur est agencé sur la carte électronique.
- il comprend des moyens de contrôle de type interface homme-machine (IHM), actionnables par l'utilisateur, par exemple un clavier ou un écran tactile de préférence connecté électroniquement à la carte de commande.
- il comprend en outre des moyens d'alimentation électrique.
- les moyens d'alimentation électrique comprennent une ou plusieurs batteries, notamment rechargeables.
- les moyens d'alimentation électrique comprennent un cordon électrique muni d'une prise de raccordement au secteur (110/220V) ou analogue.
- il comprend en outre une coque ou carcasse externe.
- le premier conduit de gaz et le quatrième conduit de gaz se réunissent, c'est-à-dire sont connectés fluidiquement l'un à l'autre, pour former un cinquième conduit de gaz ou conduit commun.
- la source d'air est en communication fluidique avec le cinquième conduit de gaz.
- la source d'air est en communication fluidique avec le cinquième conduit de gaz via un sixième conduit de gaz véhiculant l'air provenant de la source d'air, typiquement une pompe à air.
- le sixième conduit de gaz comprend un troisième capteur de débit.
- le cinquième conduit de gaz et le sixième conduit de gaz se réunissent, c'est-à-dire sont raccordés fluidiquement l'un à l'autre, pour former un septième conduit de gaz ou conduit principal.
- le septième conduit de gaz comprenant un deuxième capteur d'oxygène et/ou un capteur de protoxyde d'azote.

Par ailleurs, l'invention concerne aussi une installation de réalisation et de fourniture de mélange gazeux comprenant :

- un appareil de fourniture de mélange gazeux selon l'invention,
- une source de gaz, tel un récipient de gaz contenant un prémélange gazeux à plusieurs constituants, de préférence un prémélange $N_2O/O_2$, ladite source de gaz étant relié fluidiquement à l'appareil pour alimenter ledit appareil en prémélange gazeux, et
- un circuit patient comprenant une interface respiratoire, tel un masque respiratoire, ledit circuit patient étant relié fluidiquement à l'appareil pour recevoir un mélange gazeux fourni par ledit appareil.

L'invention va maintenant être mieux comprise grâce à la description détaillée suivante, faite à titre illustratif mais non limitatif, en référence aux figures annexées parmi lesquelles :

[Fig. 1] schématise un mode de réalisation d'une installation de réalisation et fourniture de mélange gazeux comprenant un appareil médical de fourniture de mélange gazeux à un patient selon l'invention, et

[Fig. 2] schématise un mode de réalisation d'un module de perméation de l'appareil de la [FIG. 1].

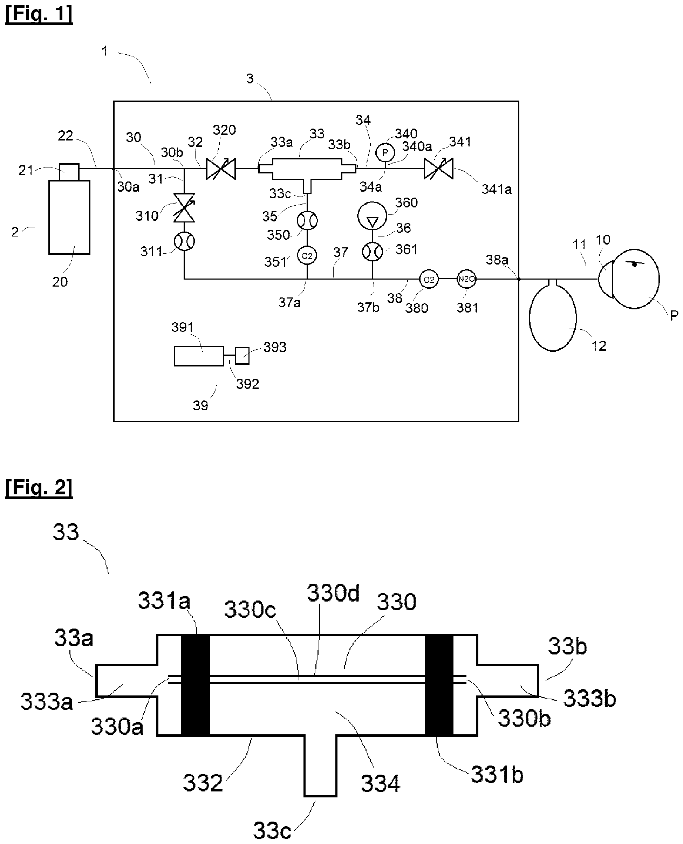

[Fig. 1] schématise un mode de réalisation d'une installation de réalisation et fourniture de mélange gazeux 1, 2, 11 comprenant un appareil médical 1 de fourniture d'un mélange gazeux à un patient, aussi appelé générateur de mélange gazeux selon l'invention, une source de gaz 2, tel un récipient de gaz, contenant un prémélange gazeux à plusieurs constituants, de préférence un prémélange $N_2O/O_2$, et un circuit patient 11.

L'appareil médical 1 de fourniture de gaz ou générateur 1 de mélange gazeux de l'invention comprend une coque ou carcasse externe 3 comprenant un conduit d'entrée 30 interne relié fluidiquement à la source de gaz 2 contenant un prémélange gazeux, ici un mélange équimolaire de qualité médicale de 50% de $N_2O$ et 50% d'$O_2$, appelé mélange $N_2O/O_2$.

La source de mélange $N_2O/O_2$ 2 est ici une bouteille de gaz 20 qui contient ce prémélange à pression élevée, par exemple jusqu'à 250 bar abs ou plus. Un régulateur de pression 21, de préférence avec moyens détendeurs de pression, c'est-à-dire un robinet à détendeur intégré ou RDI, est monté en sortie de la bouteille 20 et délivre du gaz à pression constante par exemple 5 bars abs, qui alimente le port d'entrée 30a du conduit d'entrée 30, de préférence via un conduit flexible 22 ou analogue.

Le générateur 1 de mélange gazeux de l'invention comprend en outre un port de sortie 38a raccordé fluidiquement au circuit patient 11 réalisant une connexion fluidique entre le port de sortie 38a du générateur 1 de mélange gazeux ternaire et une interface respiratoire 10, par exemple un masque respiratoire facial, servant à fournir un mélange gazeux respiratoire à un patient P, à savoir un mélange ternaire contenant essentiellement $N_2O$, $O_2$ et $N_2$ ; les autres espèces gazeuses éventuellement présentes étant considérés comme négligeables.

Le gaz fourni par la source 2 de mélange $N_2O/O_2$ chemine dans le générateur 1 de mélange gazeux, entre le port d'entrée 30a et le port de sortie 38a, au sein d'un circuit interne de gaz comprenant plusieurs conduits de gaz 30, 31, 32, 35, 37, comme expliqué ci-après.

Le circuit patient 11 comprend un réservoir de gaz 12 qui sert de réserve de gaz respiratoire au patient P.

L'alimentation constante en gaz fourni par le générateur 1, c'est-à-dire le débit de gaz souhaité, est réglée par l'utilisateur, par exemple un médecin, pour répondre à la ventilation minute du patient P, par exemple un débit gazeux de 10 L/min.

Lorsque le patient P respire (i.e. en phase d'inspiration), le réservoir de gaz 12 satisfait la demande instantanée dudit patient P en lui fournissant la quantité de gaz respiratoire dont il a besoin, alors que lorsque le patient P expire (i.e. en phase d'expiration), le réservoir de gaz 12 est rempli à nouveau par du gaz frais provenant du générateur 1.

Par ailleurs, il est à souligner que tous les éléments électromécaniques du générateur 1 de l'invention, telles les électrovannes, sont alimentés et commandés par une unité de commande 39, aussi appelée unité de pilotage ou moyens de pilotage, laquelle comprend typiquement des moyens de contrôle 393 de type interface homme-machine (IHM), qui sont actionnables par l'utilisateur, par exemple un clavier ou un écran tactile, qui est par ailleurs connecté électroniquement à une carte de commande 391, via une connexion électrique 392 adaptée.

La carte de commande 391 est conçue ou configurée pour alimenter les différents composants électromécaniques du générateur de mélange 1. Elle intègre une unité de contrôle, typiquement un (ou plusieurs) microcontrôleur, permettant de commander et/ou analyser les signaux des différents composants électromécaniques du générateur 1, en particulier une ou des vannes, capteurs...

Par ailleurs, le conduit d'entrée 30 du générateur 1 de mélange gazeux de l'invention se divise, à partir d'un embranchement 30b, en deux conduits distincts, à savoir un premier conduit 31 et un deuxième conduit 32 qui sont en communication fluidique avec le conduit d'entrée 30.

Le premier conduit 31 comprend, agencés en série, une première électrovanne proportionnelle 310 et un premier capteur de débit 311 définissant ensemble un contrôleur de débit massique. Ils sont alimentés et contrôlés par la carte de commande 391 et pilotés le microcontrôleur de la carte de commande 391.

En réponse à une action de l'utilisateur via les moyens de contrôle 393, le microcontrôleur 394 peut déterminer une consigne de débit Q et contrôler l'électrovanne proportionnelle 310 de manière à ce que le débit circulant dans le premier conduit 31, mesuré par le capteur de débit 311, soit égal à ladite consigne de débit Q.

Par exemple, on peut choisir le contrôleur de débit massique comprenant une première électrovanne proportionnelle 310 et un premier capteur de débit 311 de la série SFC disponibles auprès de la société Sensirion.

De façon analogue, une deuxième électrovanne proportionnelle 320 est agencée dans le deuxième conduit 32 qui alimente en gaz, le port d'alimentation 33a d'un module de séparation de gaz par perméation, aussi appelé aussi module de perméation 33.

Comme représenté sur la [Fig. 2], le module de perméation 33 de l'appareil 1 comprend une enveloppe 332 externe formant une carcasse en matériau rigide, par exemple en acier ou en polymère, par exemple de type PEEK.

L'enveloppe externe 332 du module de perméation 33 présente trois ports, à savoir le port d'alimentation 33a par lequel arrive le mélange gazeux d'alimentation provenant du deuxième conduit 32, un port de rétentat 33b d'où une partie du gaz d'alimentation ressort du module de perméation 33 (i.e. en étant retenu, c'est-à-dire sans avoir perméé), et un port de perméat 33c d'où une partie du gaz ayant perméé ressort du module de perméation 33.

Le port d'alimentation 33a débouche dans une première chambre 333a du module de perméation 33, aussi appelée chambre amont ou chambre d'alimentation. Le module de perméation 33 comprend par ailleurs une deuxième chambre 333b, aussi appelée chambre aval ou chambre de rétentat, comprenant le port de rétentat 33b, et une troisième chambre 334, aussi appelée chambre intermédiaire ou chambre de perméat, comprenant le port de perméat 33c.

A l'intérieur du module de perméation 33 se trouvent agencées des fibres creuses 330. Chaque fibre creuse 330 présente un canal interne ou lumen 330c dans lequel le mélange de gaz peut circuler, et est délimitée par une membrane périphérique 330d, c'est-à-dire constituant la paroi périphérique de chaque fibre creuse 330.

Chaque fibre creuse 330 a donc une structure générale tubulaire et forme ou constitue une membrane de séparation par perméation. Plus précisément, la membrane périphérique 330d comprend une paroi formée d'une couche mince de matériau, typiquement un matériau à base de silicone, tel que le PDMS, de quelques microns d'épaisseur.

Chaque fibre creuse 330 est mécaniquement maintenue par des scellements 331a, 331b, par exemple des bouchons ou analogues, assurant une étanchéité parfaite entre les première, deuxième et troisième chambres 333a, 333b et 334 du module de perméation 33. Les scellements peuvent être par exemple en polyuréthane.

Le mélange gazeux provenant du deuxième conduit 32 et alimentant la chambre 333a du module de perméation 33 passe ensuite dans le lumen 330c de chaque fibre creuse 330 en y entrant par une extrémité proximale 330a en communication fluidique avec la chambre amont 333a du module de perméation 33.

La composition des fibres creuses 330, à savoir sa membrane périphérique 330d, est choisie pour permettre de séparer sélectivement par perméation une (ou plusieurs) espèce(s) ou composé(s) gazeux présent(s) dans le mélange gazeux d'alimentation circulant dans le lumen de ladite fibre creuse 330.

Dit autrement, une molécule donnée peut traverser par perméation la membrane 330d de chaque fibre 330 et donc passer du canal interne ou lumen 330c de chaque fibre creuse 330 à la troisième chambre 334 du module de perméation 33, avant d'en sortir par le port de perméat 33c. Le nombre de molécules pénétrant dans chaque fibre creuse 330 et traversant la membrane 330d dépend des propriétés de cette membrane externe 330d et du gradient de pression existant entre le canal interne 330c de la fibre creuse 330 et la troisième chambre 334 du module de perméation 33. Plus ce gradient de pression est important, plus facilement la molécule est capable de traverser la membrane 330d de chaque fibre 330.

La membrane 330d affiche également une sélectivité plus élevée pour un composé du mélange, par exemple pour le protoxyde d'azote, de sorte de favoriser la perméation de ce composé gazeux par rapport aux autres. Par exemple, la sélectivité $N_2O/O_2$ du PDMS est de 8 :1, c'est-à-dire que le taux de perméation des molécules de $N_2O$ à travers la membrane 330d est 8 fois plus élevé que celui des molécules de $O_2$. Le PDMS convient donc à une séparation par perméation des molécules de $N_2O$ d'un mélange contenant les composés $O_2$ et $N_2O$. Bien entendu, d'autres matériaux que le PDMS peuvent être utilisés pour former la membrane 330d de chaque fibre creuse 330 dès lors que leur sélectivité est adéquate, c'est-à-dire convient à la séparation désirée.

De là, en supposant que 1 unité de gaz d'alimentation (e.g. mélange équimolaire 50% $N_2O$/50% $O_2$) entre dans le module de perméation 33 et qu'un gradient de pression existe entre le lumen 330c de chaque fibre creuse 330 et la troisième chambre 334, une partie $\beta$ du gaz d'alimentation va traverser la membrane externe 330d, c'est-à-dire le perméat. Le reste, i.e. le rétentat, qui est égal à (1- $\beta$), va sortir de chaque fibre creuse 330 par son extrémité distale 330b. Du fait de la sélectivité plus élevée de la membrane externe 330d pour les espèces $N_2O$, le débit de perméat $\beta$ est enrichi en $N_2O$ (i.e. jusqu'à environ 90% de $N_2O$) qui traverse plus facilement la membrane externe 330d, tandis que le débit de rétentat (1- $\beta$) est enrichi en $O_2$.

Préférentiellement, le module de perméation 33 est constitué de plusieurs fibres creuses 330, avantageusement plusieurs centaines ou plusieurs milliers de fibres 330, voire des centaines de milliers de fibres 330, agencées en parallèles les unes des autres. Utiliser un grand nombre de fibres 330 permet d'augmenter la capacité de séparation et d'obtenir des débits importants, notamment le perméat ($\beta$), typiquement un débit de perméat allant jusqu'à 10 L/min, voire même supérieur. De tels module de perméation 33 sont disponibles auprès de la société Porogen.

Comme détaillé en [Fig. 1], le port de rétentat 33b du module de perméation 33 est connecté fluidiquement à un troisième conduit 34 comprenant une troisième électrovanne proportionnelle 341. Un capteur de pression 340 est également prévu entre le port de rétentat 33b et la troisième électrovanne proportionnelle 341, lequel est fluidiquement connecté au troisième conduit 34 via un conduit de dérivation 340a. L'orifice de sortie 341a de l'électrovanne proportionnelle 341 débouche à l'atmosphère ambiante et permet donc d'y évacuer le gaz de rétentat provenant du module de séparation 33, par exemple de l'oxygène dans le cas du mélange équimolaire $N_2O/O_2$ susmentionné.

La plage de mesure du capteur de pression 340 est comprise entre 0 et 7 bars abs. On peut utiliser par exemple le capteur de pression référencé MS5803-07BA disponible auprès de la société TE Connectivity.

De manière similaire, le port de perméat 33c du module de perméation 33 est connecté à un quatrième conduit 35 comprenant un deuxième capteur de débit 350, de préférence un capteur de débit massique, par exemple un capteur de la série SFM disponible auprès de la société Sensirion, et un premier capteur d'oxygène 351 capable de détecter des concentrations d'O₂ comprises entre 0 et 100%, par exemple un capteur de type électrochimique, e.g. série OOM de la société Envitec, ou de type paramagnétiques, e.g. série Paracube de la société Hummingbird.

Le quatrième conduit 35 recueille donc le gaz ayant perméé au travers des fibres creuses 330, en particulier un gaz riche en $N_2O$ dans le cas susmentionné d'un mélange équimolaire $N_2O/O_2$.

Comme visible en [Fig. 1], le quatrième conduit 35 vient se raccorder fluidiquement au premier conduit 31 (en 37a) en aval du premier capteur de débit 311 pour former un conduit commun ou cinquième conduit 37 alimenté par les débits gazeux respectifs provenant des premier et quatrième conduits 31, 35.

Par ailleurs, une source d'air 360, telle une pompe à air, par exemple une pompe de la série 1610 disponible auprès de la société Thomas, aspire de l'air ambiant et le délivre dans un sixième conduit, appelé conduit d'aspiration 36, comprenant un troisième capteur de débit 361, de préférence un capteur de débit massique. Le conduit 36 est raccordé fluidiquement (en 37b) au conduit commun ou cinquième conduit 37.

En aval du site de raccordement 37b, se trouve un septième conduit ou conduit principal 38 dans lequel circule le mélange de gaz final, contenant les différents composés gazeux aux teneurs désirées provenant des cinquième et sixième conduits 37, 36, soit un mélange de $N_2O/O_2$, d'$O_2$ et d'air, respectivement, par exemple.

Afin de s'assurer de la bonne composition du mélange de gaz circulant dans le conduit principal 38, qui est composé de $N_2O$, $O_2$ et $N_2$ par exemple, et acheminé vers le patient P via le circuit patient 11, on prévoit deux capteurs supplémentaires, à savoir un deuxième capteur d'oxygène 380, similaire au premier capteur d'oxygène 351, et un capteur de protoxyde d'azote 381. Le capteur de protoxyde d'azote 381 est également capable de détecter une large plage de concentrations de $N_2O$, par exemple entre 0 et 100%. On peut utiliser par exemple le capteur référencé IRMA AX + disponible auprès de la société Phaseln.

En outre, l'appareil médical 1 de fourniture de gaz ou générateur 1 de mélange gazeux de l'invention comprend en outre des moyens d'alimentation électrique (non représentés), par exemple une ou plusieurs batteries, notamment rechargeables, et/ou un cordon électrique muni d'une prise de raccordement au secteur (110/220V) ou analogue.

Le fonctionnement de l'appareil de fourniture de gaz 1 ou générateur de mélange gazeux de l'invention est le suivant pour un prémélange gazeux formé de 50% de $O_2$ et 50% de $N_2O$ (% mol).

Un opérateur autorisé à initier la thérapie, tel un personnel soignant, utilise les moyens de contrôle 393 pour régler ou ajuster :

- un débit continu, $Q_{TOTAL}$, qui correspond à la ventilation minute du patient et délivré audit patient via le circuit patient 11 et l'interface respiratoire 10. Ce débit continu correspond au débit final qui doit être généré par le générateur de mélange 1 de l'invention.
- et une composition finale souhaitée pour un mélange gazeux ternaire à fournir au patient P, ladite composition finale souhaitée étant définie par des teneurs en protoxyde d'azote $C_{N2O}$ et en oxygène $C_{O2}$, le reste étant essentiellement de l'azote $N_2$.

On considère par ailleurs que:

- $Q_{KAL}$ : est le débit du gaz circulant dans le premier conduit 31 provenant de la source 2 de prémélange, i.e. 50% $N_2O$ et 50% $O_2$,
- $Q_{PERM}$ : est le débit circulant dans le quatrième conduit 35, issu du port perméat 33c du module de perméation 33, à une concentration donnée en $N_2O$, $C_{N2OP}$, qui se rapporte à une concentration en $O_2$ $C_{O2P} = (1-C_{N2OP})$
- $Q_{AIR}$ : est le débit d'air ambiant injecté dans le conduit 36 par la pompe 360 ayant une composition de l'ordre de 80% de $N_2$ et de 20% d'$O_2$.

La distribution des débits $Q_{KAL}$, $Q_{PERM}$ et $Q_{AIR}$ (en L/min) obéit alors aux équations suivantes:

[Math 1]

$$Q_{KAL} = \frac{5.(1 - C_{O2P}).(C_{N2O} + C_{O2} - 0.2) - 4C_{N2O}}{2.(1 - 2.C_{O2P})} . Q_{TOTAL}$$

[Math 2]

$$Q_{PERM} = \frac{(3 \cdot C_{N2O} - 5 \cdot C_{O2} + 1)}{4 \cdot (1 - 2 \cdot C_{O2P})} \cdot Q_{TOTAL}$$

[Math 3]

$$Q_{AIR} = \frac{(1 - C_{N2O} - C_{O2})}{0.8} \cdot Q_{TOTAL}$$

Dans cet exemple :

- $Q_{TOTAL}$ est réglé à 10 L / min,
- La concentration souhaitée en $N_2O$, $C_{N2O}$, est de 40%
- La concentration souhaitée en $O_2$, $C_{O2}$, est de 30%.

Ainsi, on a :

[Math 4]

$$Q_{KAL} = \frac{25 \cdot (1 - C_{O2P}) - 16}{2 \cdot (1 - 2 \cdot C_{O2P})}$$

[Math 5]

$$Q_{PERM} = \frac{7}{4 \cdot (1 - 2 \cdot C_{O2P})}$$

[Math 6]

$$Q_{AIR} = 3.75$$

Comme décrit précédemment, la source de prémélange 2 comprend un régulateur de pression 21 qui fournit une alimentation en pression constante en amont des électrovannes proportionnelles 310 et 320 (i.e. dans les premier et deuxième conduits 31, 32), par exemple de l'ordre de 5 bars abs.

Afin de générer la composition de mélange souhaitée, le microcontrôleur 394 pilote la vanne proportionnelle 320 en ouverture, ce qui amène le gaz circulant dans le deuxième conduit 32 à pénétrer dans le module de perméation 33, via son port d'alimentation 33a. Une partie β du débit entrant est déviée vers le port de perméat 33c (après avoir traversé la membrane 330d de la fibre creuse 330) tandis que le complément (1- β) de ce même débit entrant sort du module de perméation 33 par son port de rétentat 33b. Le rapport entre les deux débits β et (1-β) est déterminé par les propriétés de la fibre creuse 330 et le gradient de pression entre le canal interne 330c de ladite fibre creuse 330 et la chambre 334 du module de perméation 33. Pour maximiser la portion β de perméat (i.e. la concentration en $N_2O$), l'électrovanne proportionnelle 341 est commandée par le microcontrôleur 394 afin de maintenir une pression au port de rétentat 33b stable. Comme le port de rétentat 33b est connecté de manière fluidique au troisième conduit 34, ceci est réalisé en mesurant la pression régnant dans le troisième conduit 34 via le capteur de pression 340. La pression reçue par le microcontrôleur 394 lui sert pour définir une commande envoyée à la vanne proportionnelle 341. Par exemple, une pression de 3 bars peut être recherchée. Considérant que le port de perméat 33c (et donc la troisième chambre 334 du module de perméation 33) est proche de la pression atmosphérique (i.e. 1 bar abs), il apparaît qu'un gradient de pression existe entre le canal interne 330c de la fibre creuse 330 et ladite troisième chambre 334, ce qui permet une séparation efficace des gaz, par exemple une concentration dans le perméat en $N_2O$, $C_{N2OP}$, égale à 75% environ, c'est-à-dire une concentration en $O_2$, $C_{O2P}$, égale à 25% environ.

Comme le port de perméat 33c est connecté de manière fluidique au quatrième conduit 35 et que celui-ci comprend un capteur d'oxygène 351, il peut être déterminé par le microcontrôleur 394 que la concentration en $O_2$ $C_{O2P}$ du gaz circulant dans le quatrième conduit 35 est effectivement de 25% environ afin d'en déduire les valeurs QKAL, QPERM : QKAL = 2,75 L/min et QPERM = 3,5 L/min.

A cet effet, le microcontrôleur 394 contrôle l'ouverture de la deuxième vanne proportionnelle 320 de manière à obtenir le débit $Q_{PERM}$ souhaité dans le quatrième conduit 35 par analyse de la mesure du capteur de débit 350.

Ainsi, QPERM est égal à 3,5 L / min.

Dans le même temps, la première électrovanne proportionnelle 310 est commandée par le microcontrôleur 394 qui coopère avec le premier capteur de débit 311 afin de de délivrer un débit constant $Q_{KAL}$ de mélange non modifié de 50% de $N_2O$, 50% d'$O_2$, ici égale à 2,75 L/min, qui circule ainsi dans le premier conduit 31.

En cas de variation mineure de la composition du perméat circulant dans le quatrième conduit 35, par exemple du fait d'un vieillissement de la membrane ou à des différences entre les lots du module de perméation 33, le premier capteur d'oxygène 351 permet de déterminer la concentration réelle $C_{O2P}$ dudit perméat pour être injecter dans le jeu d'équation permettant de déterminer les débits QPERM et QKAL.

Le débit circulant dans le cinquième conduit 37 est donc la somme des débits circulant dans le premier conduit 31 (QKAL) et le quatrième conduit 35 (QPERM) qui se réunissent au niveau de l'embranchement 37a.

Afin d'atteindre la composition souhaitée, la pompe 360 est actionnée par le microcontrôleur 394 de sorte que le troisième capteur de débit 361 mesure le débit $Q_{AIR}$ souhaité, ici 3,75 L/min. Ce débit, circulant dans le sixième conduit 36, se mélange (en 37b) au débit transitant dans le cinquième conduit 37 pour générer un débit total $Q_{TOTAL}$ dans le conduit principal 38, qui est égal à celui défini par l'opérateur.

Par ailleurs les capteurs d'oxygène 380 et de protoxyde d'azote 381 renseignent le microcontrôleur 394 de la composition du mélange afin de s'assurer qu'elle est bien celle définie par l'utilisateur, c'est-à-dire ici que la concentration en $O_2$ $C_{O2}$ est de 30% et celle de $N_2O$ $C_{N2O}$ de 40%. Dans un tel cas, le mélange circulant dans le conduit principal 38 sort du générateur de mélange 1 via le port de sortie 38a pour être délivré au patient P via le circuit patient 11, le réservoir de gaz 12 et l'interface 10, comme expliqué ci-dessus.

En cas de différence trop importante de la concentration en $O_2$ $C_{O2}$ et de celle de $N_2O$ $C_{N2O}$ par rapport à la consigne réglée par l'utilisateur, le microcontrôleur peut déterminer de stopper la thérapie et d'informer l'utilisateur au moyen de signaux sonores et/ou visuels, c'est-à-dire déclencher une (ou des) alarme.

Compte tenu de l'ensemble des équations, que différents débits $Q_{TOTAL}$ et différentes concentrations de $N_2O$ ou d'$O_2$ peuvent être réglées par l'utilisateur.

Plus spécifiquement, en maintenant une concentration en $O_2$ à 30% et un débit total $Q_{TOTAL}$ à 10 L/min, on peut déterminer les débits suivants :

- concentration en $N_2O$ = 40% :
  $Q_{KAL}$ = 2,75 L/min; $Q_{PERM}$ = 3,5 L/min; $Q_{AIR}$ = 3,75 L/min.
- concentration en $N_2O$ = 50% :
  $Q_{KAL}$ = 2,5 L/min; $Q_{PERM}$ = 5 L/min; $Q_{AIR}$ = 2. 5 L/min.
- concentration en $N_2O$ = 30%:
  $Q_{KAL}$ = 3 L/min; $Q_{PERM}$ = 2 L/min; $Q_{AIR}$ = 5 L/min.

En d'autres termes, il est possible de définir une concentration différente en $N_2O$ en fonction des besoins du patient P et de générer un mélange ternaire maintenant le niveau d'oxygène à une teneur définie.

L'appareil et l'installation de fourniture de mélange gazeux selon l'invention sont particulièrement adaptés au traitement à leur domicile de patients souffrant de douleurs chroniques.

**Revendications**

**1.** Appareil (1) de fourniture d'un mélange gazeux à un patient comprenant :

- un conduit d'entrée de gaz (30) comprenant un orifice d'entrée de gaz (30a) se divisant en un premier conduit de gaz (31) et en un deuxième conduit de gaz (32),
- au moins un module de perméation (33) agencé sur le deuxième conduit de gaz (32), ledit module de perméation (33) comprenant un port d'alimentation (33a) en communication fluidique avec le deuxième conduit de gaz (32), un port de rétentat (33b) et un port de perméat (33c),
- un troisième conduit de gaz (34) en communication fluidique avec le port de rétentat (33b) du module de perméation (33),
- un quatrième conduit de gaz (35) en communication fluidique avec le port de de perméat (33c) du module de perméation (33), et venant se raccorder fluidiquement audit premier conduit de gaz (31) et
- une source d'air (360) en communication fluidique avec le premier conduit de gaz (31) et le quatrième conduit de gaz (35).

2. Appareil (1) selon la revendication précédente, **caractérisé en ce que** le module de perméation (33) comprend une pluralité de fibres creuses (330).

3. Appareil (1) selon l'une des revendications précédentes, **caractérisé en ce que** le premier conduit de gaz (31) comprend une première électrovanne proportionnelle (310) et un premier capteur de débit (311).

4. Appareil (1) selon la revendication 3, **caractérisé en ce que** le deuxième conduit de gaz (32) comprend une deuxième électrovanne proportionnelle (320).

5. Appareil (1) selon les revendications 3 et 4, **caractérisé en ce que** le troisième conduit de gaz (34) comprend un capteur de pression (340) et une troisième électrovanne proportionnelle (341).

6. Appareil (1) selon la revendication 3, **caractérisé en ce que** le quatrième conduit de gaz (35) comprend un deuxième capteur de débit (350) et un premier capteur d'oxygène (351).

7. Appareil (1) selon l'une des revendications précédentes, **caractérisé en ce que** la source d'air (360) comprend une pompe à air reliée fluidiquement à l'atmosphère.

8. Appareil (1) selon l'une des revendications précédentes, **caractérisé en ce qu'**il comprend en outre une unité de commande (39) à microcontrôleur.

9. Appareil (1) selon l'une des revendications précédentes, **caractérisé en ce que** le module de perméation (33) comprend :

   - un port d'alimentation en gaz (33a) en communication fluidique avec le deuxième conduit (32),
   - un port de rétentat (33b) en communication fluidique avec le troisième conduit de gaz (34), et
   - un port de perméat (33c) en communication fluidique avec le quatrième conduit de gaz (35).

10. Appareil (1) selon les revendications 3 et 6, **caractérisé en ce que** le premier conduit de gaz (31) et le quatrième conduit de gaz (35) se réunissent pour former un cinquième conduit de gaz (37), et la source d'air (360) est en communication fluidique avec le cinquième conduit de gaz (37), via un sixième conduit de gaz (36) comprenant un troisième capteur de débit (361).

11. Appareil (1) selon la revendication 6 et 10, **caractérisé en ce que** le cinquième conduit de gaz (37) et le sixième conduit de gaz (36) se réunissent pour former un septième conduit de gaz (38), ledit septième conduit de gaz (38) comprenant un deuxième capteur d'oxygène (380) et un capteur de protoxyde d'azote (381).

12. Installation (1, 2, 11) de réalisation et fourniture de mélange gazeux (1, 2, 11) comprenant :

   - un appareil (1) de fourniture de mélange gazeux selon l'une des revendications précédentes,
   - une source de gaz (2) contenant un prémélange gazeux à plusieurs constituants, de préférence un prémélange $N_2O/O_2$, ladite source de gaz (2) étant relié fluidiquement à l'appareil (1) pour alimenter ledit appareil (1) en prémélange gazeux, et
   - un circuit patient (11) comprenant une interface respiratoire (10), ledit circuit patient (11) étant relié fluidiquement à l'appareil (1) pour recevoir un mélange gazeux fourni par ledit appareil (1).

**Revendications modifiées conformément à la règle 137(2) CBE.**

1. Appareil (1) de fourniture d'un mélange gazeux à un patient comprenant :

   - un conduit d'entrée de gaz (30) comprenant un orifice d'entrée de gaz (30a) se divisant en un premier conduit de gaz (31) et en un deuxième conduit de gaz (32),
   - au moins un module de perméation (33) agencé sur le deuxième conduit de gaz (32), ledit module de perméation (33) comprenant un port d'alimentation (33a) en communication fluidique avec le deuxième conduit de gaz (32), un port de rétentat (33b) et un port de perméat (33c), et
   - un troisième conduit de gaz (34) en communication fluidique avec le port de rétentat (33b) du module de perméation (33),

   **caractérisé en ce qu'**il comprend en outre:

   - un quatrième conduit de gaz (35) en communication fluidique avec le port de de perméat (33c) du module de perméation (33), et venant se raccorder fluidiquement audit premier conduit de gaz (31) et
   - une source d'air (360) en communication fluidique avec le premier conduit de gaz (31) et le quatrième conduit de gaz (35).

2. Appareil (1) selon la revendication précédente, **caractérisé en ce que** le module de perméation (33) comprend une pluralité de fibres creuses (330).

3. Appareil (1) selon l'une des revendications précédentes, **caractérisé en ce que** le premier conduit de gaz (31) comprend une première électrovanne proportionnelle (310) et un premier capteur de débit (311).

4. Appareil (1) selon la revendication 3, **caractérisé en ce que** le deuxième conduit de gaz (32) comprend une deuxième électrovanne proportionnelle (320).

5. Appareil (1) selon les revendications 3 et 4, **caractérisé en ce que** le troisième conduit de gaz (34) comprend un capteur de pression (340) et une troisième électrovanne proportionnelle (341).

6. Appareil (1) selon la revendication 3, **caractérisé en ce que** le quatrième conduit de gaz (35) comprend un deuxième capteur de débit (350) et un premier capteur d'oxygène (351).

7. Appareil (1) selon l'une des revendications précédentes, **caractérisé en ce que** la source d'air (360) comprend une pompe à air reliée fluidiquement à l'atmosphère.

8. Appareil (1) selon l'une des revendications précédentes, **caractérisé en ce qu'**il comprend en outre une unité de commande (39) à microcontrôleur.

9. Appareil (1) selon l'une des revendications précédentes, **caractérisé en ce que** le module de perméation (33) comprend :

   - un port d'alimentation en gaz (33a) en communication fluidique avec le deuxième conduit (32),
   - un port de rétentat (33b) en communication fluidique avec le troisième conduit de gaz (34), et
   - un port de perméat (33c) en communication fluidique avec le quatrième conduit de gaz (35).

10. Appareil (1) selon les revendications 3 et 6, **caractérisé en ce que** le premier conduit de gaz (31) et le quatrième conduit de gaz (35) se réunissent pour former un cinquième conduit de gaz (37), et la source d'air (360) est en communication fluidique avec le cinquième conduit de gaz (37), via un sixième conduit de gaz (36) comprenant un troisième capteur de débit (361).

11. Appareil (1) selon la revendication 6 et 10, **caractérisé en ce que** le cinquième conduit de gaz (37) et le sixième conduit de gaz (36) se réunissent pour former un septième conduit de gaz (38), ledit septième conduit de gaz (38) comprenant un deuxième capteur d'oxygène (380) et un capteur de protoxyde d'azote (381).

12. Installation (1, 2, 11) de réalisation et fourniture de mélange gazeux (1, 2, 11) comprenant :

- un appareil (1) de fourniture de mélange gazeux selon l'une des revendications précédentes,
- une source de gaz (2) contenant un prémélange gazeux à plusieurs constituants, de préférence un prémélange $N_2O/O_2$, ladite source de gaz (2) étant relié fluidiquement à l'appareil (1) pour alimenter ledit appareil (1) en prémélange gazeux, et
- un circuit patient (11) comprenant une interface respiratoire (10), ledit circuit patient (11) étant relié fluidiquement à l'appareil (1) pour recevoir un mélange gazeux fourni par ledit appareil (1).

[Fig. 1]

[Fig. 2]

**Europäisches Patentamt**
**European Patent Office**
**Office européen des brevets**

## RAPPORT DE RECHERCHE EUROPEENNE

Numéro de la demande

EP 20 15 9190

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 96/37176 A1 (KOTLIAR IGOR K [US]) 28 novembre 1996 (1996-11-28) * page 6, ligne 18 - page 7, ligne 23; figures 1-4 * ----- | 1-12 | INV. A61M16/00 A61M16/10 A61M16/12 A61M16/20 B01D53/00 B01D53/22 |
| A | WO 2004/073780 A1 (VOLGYESI GEORGE A [CA]) 2 septembre 2004 (2004-09-02) * figures 1-5 * ----- | 1-12 | |
| A | GB 2 553 790 A (SPORTING EDGE UK LTD [GB]) 21 mars 2018 (2018-03-21) * figures 1-3 * ----- | 1-12 | |

| | DOMAINES TECHNIQUES RECHERCHES (IPC) |
|---|---|
| | A61M B01D |

Le présent rapport a été établi pour toutes les revendications

| Lieu de la recherche | Date d'achèvement de la recherche | Examinateur |
|---|---|---|
| Munich | 17 avril 2020 | Louarn, Arzhur |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**

EP 20 15 9190

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-04-2020

| Document brevet cité au rapport de recherche | | Date de publication | Membre(s) de la famille de brevet(s) | | Date de publication |
|---|---|---|---|---|---|
| WO 9637176 | A1 | 28-11-1996 | US | 5850833 A | 22-12-1998 |
| | | | WO | 9637176 A1 | 28-11-1996 |
| WO 2004073780 | A1 | 02-09-2004 | US | 2004163706 A1 | 26-08-2004 |
| | | | WO | 2004073780 A1 | 02-09-2004 |
| GB 2553790 | A | 21-03-2018 | GB | 2553790 A | 21-03-2018 |
| | | | WO | 2018051053 A1 | 22-03-2018 |

EPO FORM P0460

Pour tout renseignement concernant cette annexe : voir Journal Officiel de l'Office européen des brevets, No.12/82